Europäisches Patentamt

European Patent Office

Office européen des brevets

(11) Veröffentlichungsnummer: **0 143 292**
**A1**

(12) **EUROPÄISCHE PATENTANMELDUNG**

(21) Anmeldenummer: **84112245.0**

(22) Anmeldetag: **11.10.84**

(51) Int. Cl.⁴: **A 61 B 5/14**

(30) Priorität: **27.10.83 DE 8330833 U**

(43) Veröffentlichungstag der Anmeldung: **05.06.85**
**Patentblatt 85/23**

(84) Benannte Vertragsstaaten: **AT BE CH DE FR GB IT LI LU NL SE**

(71) Anmelder: **Ballies, Uwe Werner, Dr., Jägersberg 7 - 9, D-2300 Kiel (DE)**

(72) Erfinder: **Ballies, Uwe Werner, Dr., Jägersberg 7 - 9, D-2300 Kiel (DE)**

(74) Vertreter: **UEXKÜLL & STOLBERG Patentanwälte, Beselerstrasse 4, D-2000 Hamburg 52 (DE)**

(54) **Blutentnahmevorrichtung.**

(57) Eine Blutentnahmevorrichtung weist ein Röhrchen (2) auf, welches ein Verschlußteil (8) und einen daran ansetzenden Kanülenstutzen (10) besitzt, auf den eine Kanüle (12) mit ihrem Kanülenkonus (14) aufsteckbar ist und bei der zwischen dem Röhrchen (2) und der Kanüle (12) ein Ventilelement (18) liegt.

Der Kanülenkonus (10) ist mit einem Außenzylinder (11) versehen.

Das Ventilelement (18) ist ein Hütchen aus flexiblem Material, welches auf den Außenzylinder (11) steckbar ist, in dieser Lage in den Kanülenkonus (14) paßt und in seinem geschlossenen Ende eine durch Dehnung freigebbare, im entlasteten Zustand hingegen geschlossene Öffnung (26) besitzt. Der Außenzylinder (11) ist länger als das Ventilelement (18).

**UEXKÜLL & STOLBERG**
PATENTANWÄLTE

BESELERSTRASSE 4
D-2000 HAMBURG 52

EUROPEAN PATENT ATTORNEYS

0143292

DR. J.-D. FRHR. von UEXKÜLL
DR. ULRICH GRAF STOLBERG
DIPL.-ING. JÜRGEN SUCHANTKE
DIPL.-ING. ARNULF HUBER
DR. ALLARD von KAMEKE

Dr. Uwe Ballies
Jägersberg 7-9

2300 Kiel

Prio:27.Okt.1983
G 83 30 833.4
(21296 hu/do)

## Blutentnahmevorrichtung

Die Erfindung betrifft eine Blutentnahmevorrichtung mit einem Röhrchen, welches einen Boden und einen daran ansetzenden Außenkonus aufweist, auf den eine Kanüle mit ihrem Innenkonus aufsteckbar ist und bei der zwischen dem Röhrchen und der Kanüle ein Ventilelement liegt.

Aus der DE-PS 24 55 631 ist bereits eine Blutentnahmevorrichtung bekannt, welche zwischen dem Blutentnahmeröhrchen und der Kanüle ein Zwischenstück aufweist. Das Zwischenstück enthält ein Ventil, das beim Einstecken der konischen Spitze des Entnahmeröhrchens in die dafür bestimmte konische Öffnung von dieser Spitze geöffnet wird, sich aber nach dem Abnehmen der Blutentnahmevorrichtung beim Herausziehen der konischen Spitze wieder schließt und somit den Austritt von Blut während des Auswechselns verhindert. Das Ventil steht dabei unter der Wirkung eines Federmittels, welches das Ventil in seiner Ruhestellung geschlossen hält. Das bekannte Zwischenstück ist so ausgebildet, daß beim Einführen der Spitze der Blutentnahmevorrichtung der Ventilkörper gegen die Wirkung des Federmittels, vorzugsweise Kunststoff-Federarme oder eine Druckfeder, in die Öffnungsstellung gebracht wird und daß dann das Blut aus der Kanüle durch das Innere des Zwischenstücks hindurch in die Spitze der Blutentnahmevorrichtung gelangen kann. Der Aufbau des Zwischenstücks ist verhältnismäßig kompliziert und erfordert wenigstens drei kompliziert geformte Teile, die außerdem noch zu dem Zwischenstück vereinigt werden müssen.

Aus der DE-OS 30 23 574 ist eine Blutentnahmevorrichtung bekannt, welche eine besonders geformte Halterung am röhrchenseitigen Ende der Kanüle aufweist, in die ein Einsatzkörper als Ventilelement einsetzbar ist. Der Einsatz-körper weist einen Innenkonus auf, welcher auf den Außenkonus des Entnahmeröhrchens steckbar ist. Kanülenseitig ist der Einsatzkörper mit einer in bezug auf die Mittellinie des Innenkonus geneigten Fläche versehen, welche von einem Blattfederelement überdeckt wird. Durch Einstecken des Röhrchenkonus wird das Blattfederelement von seiner Auflagefläche abgehoben, so daß das Ventilelement geöffnet ist und Blut durch die Kanüle in das Entnahmeröhrchen strömen kann. Wird das Röhrchen jedoch aus dem Einsatzkörper gezogen, so drückt der Blutstrom das Blattfederelement auf die geneigte Fläche und verschließt die Kanüle. Dieses bekannte Zwischenstück ist ebenfalls verhältnismäßig kompliziert geformt und erfordert ähnlich kompliziert geformte Kanülen.

Schließlich ist aus dem DE-GM 80 16 927 ein Adapter für den Übergang von einem Blutentnahmeröhrchen zu einer Kanüle bekannt, welcher an einem Ende eines zylindrischen Gehäuses einen angeformten Außenkonus für das Aufstecken einer handelsüblichen Kanüle aufweist. Das dem Außenkonus gegenüberliegende Ende des Gehäuses ist offen und dient zum Einstecken des Entnahmeröhrchens. Der Außenkonus des Adapters ist mit einer Längsbohrung versehen, in der eine Hohlnadel mit einer darübergeschobenen Hülse aus elastischem Material als Ventil steckt. Bei der Blutentnahme wird ein Aufnahmestutzen des Entnahmeröhrchens in das Adaptergehäuse eingeführt und schiebt die Hülse auf die Hohlnadel, wobei deren freies Ende sowohl die Hülse als auch eine auf dem Aufnahmestutzen des Röhrchens vorgesehene Kappe durchsticht.

Sobald die Blutentnahmevorrichtung aus dem Adapter herausgezogen wird, verschließt die Hülse wieder das freie Ende der Hohlnadel und verhindert dadurch den Austritt von Blut aus dem punktierten Gefäß.

Es ist Aufgabe der Erfindung, die Blutentnahmevorrichtung der eingangs genannten Art dahingehend zu verbessern, daß ihr Ventilelement einen noch einfacheren Aufbau hat, um damit der Allgemeinheit eine Blutentnahmevorrichtung mit Ventilelement für die mehrfache Blutentnahme zu einem Herstellungspreis zur Verfügung zu stellen, der etwa einer Blutentnahmevorrichtung ohne Ventilelement entspricht und im Rahmen einer normalen Handhabung bei der Blutentnahme keine gesonderten Handgriffe erfordert.

Zur Lösung dieser Aufgabe dient eine Vorrichtung der eingangs erwähnten Art, welche dadurch gekennzeichnet ist, daß der Kanülenkonus einen Außenzylinder aufweist; daß das Ventilelement ein Hütchen aus flexiblem Material ist, welches auf den Außenzylinder steckbar ist, in dieser Lage in den Kanülenkonus paßt und in seinem geschlossenen Ende eine durch Dehnung freigebbare, im entlasteten Zustand hingegen geschlossene Öffnung besitzt; und daß der Außenzylinder länger als das Ventilelement ist.

Dadurch wird erreicht, daß das flexible Hütchen vor der Blutentnahme geschlossen ist und erst durch das vollständige Einschieben des Kanülenstutzens des Entnahmeröhrchens im Bereich seiner Öffnung für den Durchtritt von Blut von der Kanüle zum Entnahmeröhrchen aufgespreizt wird. Beim Herausziehen des Röhrchen-Außenzylinders wird der Boden des Hütchens entlastet und aufgrund des flexiblen Hütchenmaterials schließt sich die Öffnung wieder, so daß der Austritt von Blut aus der Kanüle verhindert wird, während das flexible Hütchen oder Ventilelement automatisch in dem Kanülenkonus verbleibt, wenn das Entnahmeröhrchen wieder aus dem Kanülenkonus herausgezogen wird. Das auf die Außenseite des Hütchens wirkende Blutvolumen neigt dazu, dieses zusammenzudrücken und unterstützt damit das Verschließen der Öffnung.

Die Öffnung ist zweckmäßigerweise ein angenähert punktförmiger Durchstich oder ein geradliniger oder sternförmiger Schnitt. Es sind jedoch auch andere Öffnungsformen denkbar, sofern nur gewährleistet ist, daß sie im entlasteten Zustand des flexiblen Hütchens dessen Boden verschließen, bei Einwirkung einer Spreizkraft jedoch eine Öffnung freigeben.

In einer bevorzugten Ausführungsform wird das flexible Ventilelement bei üblicher Handhabung automatisch in den Innenkonus einer Kanülenhalterung eingesetzt und weist an seinem Rand widerhakenartige Vorsprünge oder Rauhigkeiten auf, die zum Eingriff mit der Wand des Innenkonus bestimmt sind. Die Vorsprünge oder Rauhigkeiten sollen bei in den Kanülenkonus eingestecktem Ventilelement verhindern, daß dieses beim Herausziehen des Entnahmeröhrchens wieder aus dem Kanülenkonus entfernt wird.

Der an den Kanülenstutzen des Entnahmeröhrchens anschließende Außenzylinder ist vorzugsweise länger als die Länge des flexiblen Hütchens, so daß das Vorderende des Außenzylinders eine Spreizkraft auf den Boden des

- 5 -

0143292

Ventilelements ausüben und dessen Öffnung aufspreizen kann.

Es wird darauf hingewiesen, daß der Kanülenstutzen an den Boden des Entnahmeröhrchens angeformt oder an einem eigens zum Verschließen des Entnahmeröhrchens vorgesehenen Verschlußteil angeordnet sein kann. Außerdem kann die Anwendung der Erfindung je nach Gegebenheit it geschlossenen oder mit geöffneten Verschlußelement erfolgen.

Die Erfindung wird im folgenden anhand von Figuren näher erläutert; es zeigen:

Figur 1   ein Ausführungsbeispiel im Längsschnitt;

Figur 2   einen vergrößerten Teilschnitt durch das Ausführungsbeispiel gemäß Figur 1 vor dem Einsetzen eines Ventilelements in einen Kanülenkonus;

Figur 3   einen vergrößerten Teilschnitt ähnlich wie Figur 2, jedoch mit in den Kanülenkonus eingesetztem Ventilelement;

Figur 4   eine Stirnansicht des Ventilelements aus Figur 1 mit einem geradlinigen Schlitz;

Figur 5   eine Stirnansicht ähnlich wie Figur 4, jedoch mit einem kreuzförmigen Schlitz;

Figur 6   eine   Endansicht   in   eine   andere   Ausführung
eines Ventilelements; und

Figur 7   einen   Längsschnitt   durch   das   Ventilelement
gemäß Figur 6.

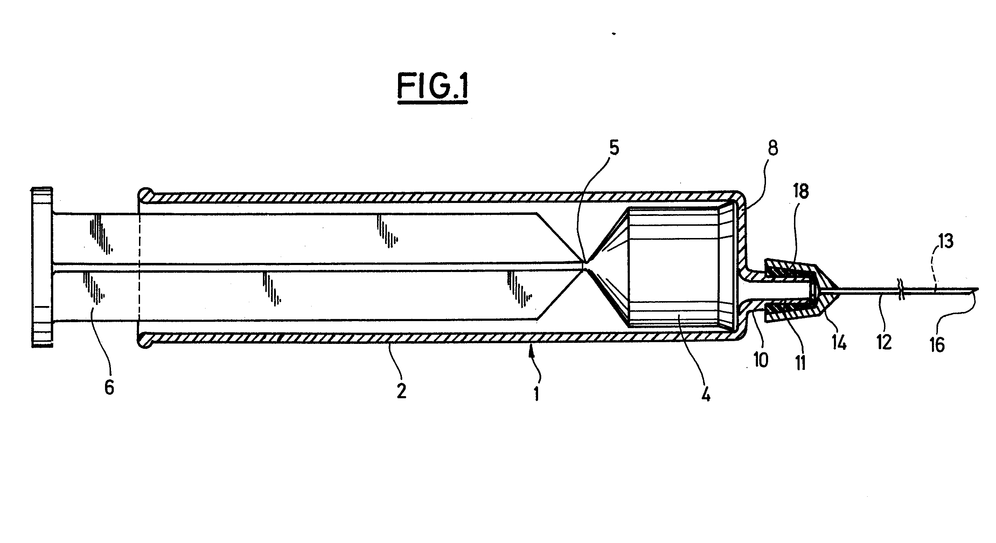

Figur 1 zeigt eine Blutentnahmevorrichtung 1 mit einem
Röhrchen 2, das an einem Ende durch einen ein Verschlußteil bildenden Boden 8 verschlossen und an seinem anderen, gegenüberliegenden Ende offen ist. An den Boden 8
setzt ein Kanülenstutzen 10 an, zweckmäßigerweise ein
sogenannter Luer-Konus, der in einen Außenzylinder 11
aufweist. In das Röhrchen 2 ist ein Kolben 4 eingeschoben, der in an sich bekannter Weise durch eine mit ihm
verbundene Kolbenstange 6 im Röhrchen 2 verschiebbar
ist und Flüssigkeit, insbesondere Blut, durch eine im
Kanülenstutzen 10 vorgesehene Durchgangsbohrung ansaugen kann. In ebenfalls an sich bekannter Weise ist die
Kolbenstange 6 über eine Sollbruchstelle 5 mit dem
Kolben 4 verbunden und kann bei am offenen Ende des
Röhrchens 2 stehendem Kolben 4 abgebrochen werden.

Auf den Außenzylinder 11 des Röhrchens 2 ist ein Ventilelement 18 in Form eines Hütchens aus flexiblem Material, vorzugsweise Kautschuk, aufgesteckt, welches in
einen Kanülenkonus 14 einer Kanüle 12 greift. Die
Kanüle 12 hat eine im allgemeinen mit einem Facettenschliff versehene Spitze 16 und weist einen Kanülenkanal 13 auf, durch den Blut nach dem Punktieren eines
Gefäßes in das Röhrchen 2 gesaugt werden kann.

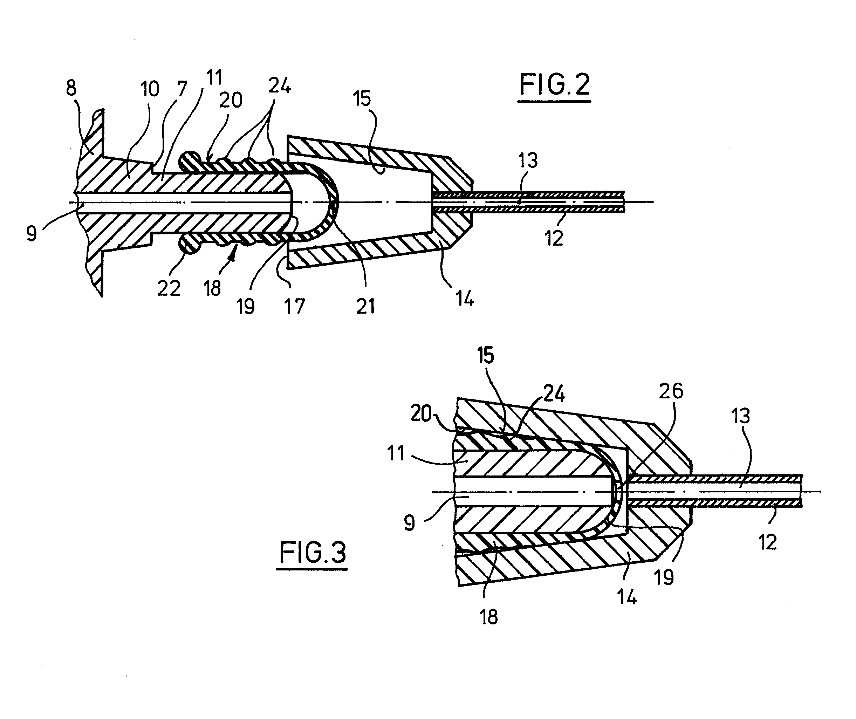

Figur 2 zeigt Einzelheiten des Kanülenstutzen 10, des Ventilelements 18 und des Kanülenkonus 14. Man erkennt, daß im Gegensatz zu herkömmlichen Entnahmeröhrchen der Luer-Konus des Kanülenstutzens 10 nicht bis zum vordersten Punkt des Röhrchens 2 verläuft, sondern über eine ringförmige Schulter 7 in den Außenzylinder 11 übergeht. Der Außenzylinder 11 weist eine Bohrung 9 auf, welche vom Vorderende 19 des Außenzylinders 11 bis zum Innenraum des Röhrchens 2 durchgängig ist und den Durchtritt von Flüssigkeit, insbesondere Blut, gestattet. Der Durchmesser der Bohrung 9 entspricht den üblichen Durchgangsbohrungen von Entnahmeröhrchen. Auf den Außenzylinder 11 ist das Ventilelement 18 als Hütchen aus flexiblem Material, vorzugsweise Kautschuk oder Kunststoff, aufgesteckt und weist an seinem geschlossenen Ende eine von einem Durchstich oder von ein oder mehreren Einschnitten 21 bzw. 23 gebildete Öffnung 26 auf. Die Öffnung 26 ist im entlasteten Zustand des Ventilelements 18 geschlossen, da sich die entsprechenden Schnittflächen dichtend einanderlegen. Auf dem Mantel 20 des Ventilelements 18 sind widerhakenartige Vorsprünge 24 oder Rauhigkeiten vorgesehen, welche dazu dienen, das in den Kanülenkonus 14 eingesteckte Ventilelement 18 im Kanülenkonus 14 durch erhöhte Reibung festzuhalten, und zwar insbesondere dann, wenn der Außenzylinder 11 aus dem Ventilelement 18 zurückgezogen wird. Am offenen Ende des hütchenartigen Ventilelements 18 ist ein Wulst 22 vorgesehen, welcher die Einsteckbewegung des Ventilelements 18 in den Kanülenkonus 14 begrenzt.

Der Kanülenkonus 14 weist einen Innenkonus 15 auf, der zweckmäßigerweise ebenfalls ein Luer-Konus ist und in den in einem nicht dargestellten Ausführungsbeispiel Vertiefungen eingeformt sind, welche zur Aufnahme der Vorsprünge 24 des Ventilelements 18 dienen und eine hierzu geeignete Form haben.

Figur 3 zeigt das von dem Röhrchen 2 vollständig in den Kanülenkonus 14 eingesteckte Ventilelement 18. Man erkennt außerdem den zur Blutentnahme vollständig in das Ventilelement 18 eingeschobenen Außenzylinder 11, dessen Vorderende 19 eine Spreizkraft auf den Boden des hütchenförmigen Ventilelements 18 ausübt und dabei den Durchstich oder Einschnitt 21 bzw. 23 zu der Öffnung 26 aufspreizt, so daß ein Strömungspfad von dem Kanülenkanal 13 durch die Öffnung 26 und durch die Bohrung 9 gebildet ist. Wie bereits erwähnt, verschließt sich die Öffnung 26 durch die Rückstellkraft des flexiblen Ventilelements 18 wieder, wenn der Außenzylinder 11 aus dem Ventilelement 18 und damit aus dem Kanülenkonus 14 zurückgezogen wird.

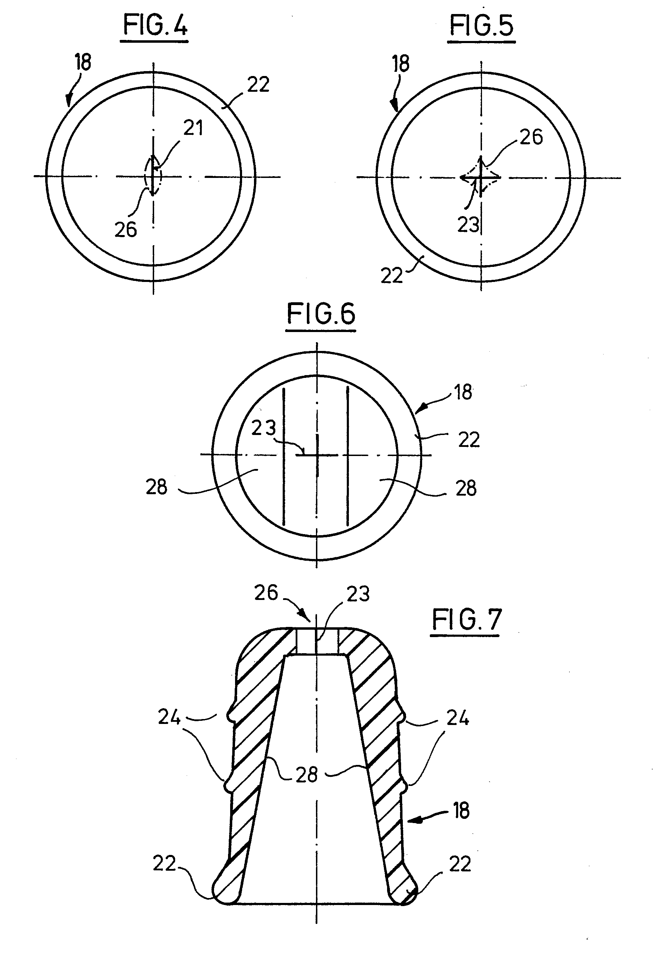

Die Figuren 4 und 5 zeigen Schnittformen für die Bildung der Öffnung 26, und zwar einen geradlinigen Radialschnitt 21 in Figur 4 und einen Kreuzschnitt 23 bei dem Ausführungsbeispiel gemäß Figur 5. Die voll ausgezogenen Linien zeigen die Schnittformen bei entlastetem Boden des Ventilelements 18 an, während die strichpunktierten Linien die Formen der aufgespreizten Öffnung 26 andeuten.

Figur 6 zeigt das Innere eines anderen Ausführungsbeispiels des Ventilelements 18, wobei wiederum gleiche Teile wie in den vorhergehenden Figuren mit gleichen Bezugszeichen versehen sind. Das Ventilelement 18 weist bei diesem Ausführungsbeispiel zwei in Richtung auf die Öffnung 26 bzw. auf den Schnitt 23 konvergierende, symmetrisch angeordnete Spreizflächen 28 auf, welche die Aufspreizwirkung des Außenzylinders 11 bei dessen Eindringen in das Ventilelement 18 verstärken, während sie andererseits das Zurückziehen des Außenzylinders 11 und damit des Röhrchens 2 aus dem Ventilelement 18 und damit aus dem Kanülenkonus 14 unterstützen. In diesem Zusammenhang wird darauf hingewiesen, daß das Ventilelement 18 nicht fest auf dem Außenzylinder 11 des Röhrchens 2 sitzen muß, sondern daß lediglich die Forderung besteht, das Ventilelement 18 mit dem Außenzylinder 11 des Röhrchens 2 in den Kanülenkonus 14 der Kanüle 12 übertragen zu können. Ist das Ventilelement 18 einmal in den Kanülenkonus 14 eingesetzt und wird es dort allein durch die Reibung zwischen seinem Mantel 20 und dem Innenkonus 15 gehalten, dann soll der Außenzylinder 11 des Röhrchens 2 lediglich zum Aufspreizen der Schnitte 21 bis 23 zu der Öffnung 26 dienen, so daß zwischen der Oberfläche des Außenzylinders 11 und der Innenwand des Ventilelements 18 keine hohe Reibungskraft vorhanden sein soll.

Das erfindungsgemäße Ventilelement 18 ist in einer bevorzugten Ausführung ein zylinderförmiges Hütchen, das über einen im Querschnitt kreisförmigen Außenzylinder 11 gesteckt wird. Der Querschnitt des Außenzylinders 11 braucht jedoch nicht kreisförmig zu sein, beispielsweise könnte er einen vieleckigen Querschnitt haben oder sogar vierkantig sein, wobei seine Ecken vorteilhafterweise abgerundet sind. Es liegt im Fachkönnen des Durchschnittsfachmanns, hier die zweckmäßigste Form auszuwählen.

## Ansprüche

1. Blutentnahmevorrichtung mit einem Röhrchen (2),
   welches ein Verschlußteil (8) und einen daran ansetzenden Kanülenstutzen (10) aufweist, auf den eine
   Kanüle (12) mit ihrem Kanülenkonus (14) aufsteckbar
   ist und bei der zwischen dem Röhrchen (2) und der
   Kanüle (12) ein Ventilelement (18) liegt,
   dadurch gekennzeichnet,
   - daß der Kanülenkonus (10) einen Außenzylinder
     (11) aufweist;
   - daß das Ventilelement (18) ein Hütchen aus flexiblem Material ist, welches auf den Außenzylinder
     (11) steckbar ist, in dieser Lage in den Kanülen-
     konus (14) paßt und in seinem geschlossenen Ende
     eine durch Dehnung freigebbare, im entlasteten Zustand hingegen geschlossene Öffnung (26) besitzt;

- und daß der Außenzylinder (11) länger als das Ventilelement (18) ist.

2. Vorrichtung nach Anspruch 1, dadurch gekennzeichnet, daß das Ventilelement (18) auf seinem äußeren Mantel (20) widerhakenartige Vorsprünge (24) oder Rauhigkeiten aufweist.

3. Vorrichtung nach Anspruch 1, dadurch gekennzeichnet, daß an dem für das Einsetzen des Außenzylinders (11) offenen Ende des Ventilelements (18) ein Wulst (22) vorgesehen ist, der bei vollständig in den Kanülen-konus (14) eingeschobenem Ventilelement (18) an dem Rand (17) des Kanülenkonus (14) anliegt.

4. Vorrichtung nach einem der Ansprüche 1 bis 3, dadurch gekennzeichnet, daß die Innenwand des Ventilelements (18) zylindrisch ist.

5. Vorrichtung nach einem der Ansprüche 1 bis 3, dadurch gekennzeichnet, daß an der Innenwand des Ventilelements (18) in Richtung auf die Öffnung (26) konvergierende Spreizflächen (28) vorgesehen sind.

6. Vorrichtung nach Anspruch 5, dadurch gekennzeichnet, daß zwei Spreizflächen (28) symmetrisch zueinander angeordnet sind.

7. Vorrichtung nach einem der Ansprüche 1 bis 6, dadurch gekennzeichnet, daß die Öffnung (26) des Ventilelements (18) von einem Durchstich oder einem Schnitt gebildet ist.

0143292

8. Vorrichtung nach Anspruch 7, dadurch gekennzeichnet, daß der Schnitt ein Radialschnitt (21), ein Kreuzschnitt (23) oder ein ähnlicher Schnitt ist, bei dem die Schnittränder im unaufgespreizten Zustand aneinanderliegen.

0143292

FIG.1

FIG.2

FIG.3

0143292

**FIG.4**

18    22
21
26

**FIG.5**

18
26
23
22

**FIG.6**

18
22
23
28    28

**FIG.7**

26    23
24    24
28
18
22    22

<table>
<tr><td colspan="2"></td></tr>
</table>

**Europäisches Patentamt**

**EUROPÄISCHER RECHERCHENBERICHT**

0143292

Nummer der Anmeldung

EP 84 11 2245

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A-2 019 219 (FRESENIUS)  *  Zusammenfassung; Figuren 1-3; Seite 2, Zeilen 13-47 * | 1-4, 6-8 | A 61 B 5/14 |
| Y | DE-A-2 531 175 (ZEPPELIN)  *  Figur 1; Seite 4, Zeilen 1-20; Seite 5, Zeile 15 - Seite 6, Zeile 3 * | 1,4,6 | |
| A | | 7,8 | |
| Y | US-A-3 212 678 (BLANCHARD)  *  Figuren 10-13,19; Spalte 6, Zeilen 25-65 * | 1,4,6 | |
| A | | 3,5 | **RECHERCHIERTE SACHGEBIETE (Int. Cl.4)**  A 61 B  A 61 M |

Der vorliegende Recherchenbericht wurde für alle Patentansprüche erstellt.

| Recherchenort | Abschlußdatum der Recherche | Prüfer |
|---|---|---|
| DEN HAAG | 04-02-1985 | Miss A.H. CHEN |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82